# EUROPEAN PATENT SPECIFICATION

(11) **EP 2 766 327 B1**
(45) Date of publication and mention of the grant of the patent: **28.09.2016**
(21) Application number: 12773296.4
(22) Date of filing: 10.10.2012
(51) Int. Cl.: C07C 2/50, C07C 2/52, C07C 4/06, C07C 5/03, C10G 1/00, C10G 45/32, C10M 107/02, C10M 109/02, C07C 13/20, C07C 13/18, C07C 9/16, C07C 9/18, C10L 1/06

(54) **PROCESS FOR PREPARING JET FUEL FROM MOLECULES DERIVED FROM BIOMASS**
VERFAHREN ZUR HERSTELLUNG EINES DÜSENTREIBSTOFFS AUF GRUNDLAGE VON AUS EINER BIOMASSE GEWONNENEN MOLEKÜLEN
PROCÉDÉ DE PRÉPARATION D'UN CARBURANT AVIATION À PARTIR DE MOLÉCULES DÉRIVÉES D'UNE BIOMASSE

(30) Priority: 11.10.2011 FR 1159153
(43) Date of publication of application: 20.08.2014
(73) Proprietor: Total Marketing Services, 92800 Puteaux (FR)
(72) Inventor: MAZURELLE, Jean, B-1630 Linkebeek (BE)
(74) Representative: Largeau, Béatrice
(86) International application number: PCT/EP2012/070035
(87) International publication number: WO 2013/053740

(56) References cited:
- WO-A1-2009/039201
- US-B1- 7 589 243
- ARNO BEHR ET AL: "Myrcene as a Natural Base Chemical in Sustainable Chemistry: A Critical Review", CHEMSUSCHEM, vol. 2, no. 12, 21 December 2009 (2009-12-21), pages 1072-1095, XP055028810, ISSN: 1864-5631, DOI: 10.1002/cssc.200900186
- HUBER GEORGE W ET AL: "Synthesis of transportation fuels from biomass: Chemistry, catalysts, and engineering", CHEMICAL REVIEWS, AMERICAN CHEMICAL SOCIETY, US, vol. 106, no. 9, 1 September 2006 (2006-09-01), pages 4044-4098, XP002490759, ISSN: 0009-2665, DOI: 10.1021/CR068360D [retrieved on 2006-06-27]

## Description

The invention relates to a process for preparing jet fuels from molecules derived from biomass.

Jet fuels consist of C₉-C₁₁ up to C₁₃-C₁₄ compounds. The cutoff points of the jet fuel fraction range between 185°C for the start point and 220-240°C for the end point. Jet fuels precursors generally also consist of such compounds presenting a maximum aromatics content of 25%.

A commercial jet fuel is obtained by mixing various fuel bases. The fuel bases are obtained in the 185-240°C distillation fractions from various crude oils. The distillation may be followed by a hydrotreatment or, in certain cases, by a softening step (Merox process, for example). Depending on the refining scheme, a kerosene fraction may also be obtained at a hydrocracker outlet. This fraction may also be incorporated into fuel bases serving for the formulation of jet fuel.

The choice of fuel bases and their relative proportions are made so that the final properties of the blend satisfy the customs specifications. By way of example, the AFQRJOS standard (issue 22) relating to A1 jet fuel gives the customs, administrative and excise specifications for this fuel.

Jet fuels are usually obtained by mixing fuel bases derived from distillation. However, these fuel bases may also be prepared via other routes, such as Fischer-Tropsch synthesis followed by a cracking step.

As a result of the increasing scarcity of fossil resources and of ever-increasing environmental concerns, the use of molecules derived from biomass is increasingly sought to replace molecules of fossil origin.

US7589243 B1 discloses jet fuel compositions comprising a mixture of limonane, farnesane, p-cymene and a petroleum-based fuel or a Fischer-Tropsch based fuel. Limonane, farnesane and p-cymene are made by hydrogenation of isoprenoid starting materials such as α-farnesene, β-farnesene and limonene.

WO2009/039201 A1 discloses methods of refining hydrocarbon feedstocks. A catalytic cracking process for cracking a terpene or a mixture of terpenes is disclosed. A fuel product with an octane rating between about 85 to 125 is obtained. A fuel component such as ethanol, jet fuel, diesel, biodiesel or gasoline can be added to the fuel product.

However, the preparation of jet fuel from molecules derived from biomass that can be used directly in the formulation of jet fuel is a veritable economical and environmental challenge.

To this end, the present invention relates to a process for preparing jet fuel that can be used starting with molecules derived from biomass. In particular, the present invention makes it possible to incorporate molecules constituting jet fuel or jet fuel precursors in the formulation of jet fuels.

A first subject of the invention is thus a process for preparing jet fuel or jet fuel precursors, i.e. hydrocarbon-based molecules with a chain length formed from 9 to 14 carbon atoms, which process includes the treatment of a charge derived from biomass, said charge comprising at least one compound chosen from:
- terpenes of formula [CH₂=C(CH₃)CH=CH₂]n, in which n is an integer of from 2 to 12, the carbon-based chain of which is linear, cyclic or branched, or
- terpenoids which are cyclic or branched terpenes as defined previously, which have been chemically modified by oxidation and/or rearrangement of the carbon backbone with an alkyl group added or removed,
said process comprising a cycloaddition step (i) followed by a hydrogenation and selective cracking step (ii).

The process according to the invention makes it possible especially to obtain products with carbon chain lengths from C9 to C14.

The cycloaddition step (i) makes it possible to react two compounds of the terpene or terpenoid family in order to obtain compounds including a naphthenic ring containing a double bond.

In particular, step (i) is a cycloaddition reaction of two compounds or of at least two compounds of the charge. Advantageously, the charge is essentially consisting of at least one compound of the terpenes or terpenoids family or mixture thereof. The terpene or terpenoid compound reacts either with itself or a terpene reacts with a terpenoid compound, preferably according to a dimerisation reaction. This reaction thus requires two compounds of the terpenes or terpenoids family in order to increase the length of chains of the latter with generation of a ring containing compound, in particular of the naphthenic type, containing a double bond.

The cycloaddition may advantageously be performed at temperatures of from 120°C to 200°C, at pressures of at least 1 Atm, preferably between 1 and 3 Atm, the reaction may be carried out during 2h to 6h. Compounds obtained according to step (i) may advantageously be obtained with yields of from 45% to 60%.

The cycloaddition may preferentially take place via a Diels-Alder reaction. However, other cycloadditions may also be envisaged.

Step (ii) of hydrogenation and selective cracking of compounds issued from step (i) enables the cleavage of a C-C bond alpha to the naphthenic ring and the saturation of the double bonds, resulting in the formation of a paraffinic compound and a naphthenic compound. The latter compound may be recycled into step (ii) and undergo a new selective cracking step in order to form a cyclohexane and a linear paraffin.

The process according to the invention also makes it possible to obtain a mixture of paraffins and/or naphthenes with a carbon chain length that is suitable for jet applications, especially presenting a C9 - C14 carbon chain length.

Step (ii) of hydrogenation and selective cracking of compounds derived from step (i) may advantageously be performed at temperatures of from 300°C° to 400°C, at pressures of at least 50 Bar, preferably between 40 and 100 Bar, the reaction preferably being carried out during 4h to 24h. Compounds obtained according to step (ii) may be obtained with yields of from 2% to 10%.

The cracking reaction of step (ii) is a catalytic cracking reaction advantageously performed under dihydrogen. In particular, the cracking reaction conditions are such that hydrogenation of the compounds takes place during the cracking reaction.

By modifying the operating conditions and the nature of the catalysts used in step (ii), it is possible to promote the cracking reaction so as to obtain molecules whose carbon chain length is from C9 to C14.

Terpenes are molecules of natural origin, produced by numerous plants, in particular conifers.

By definition, terpenes (also known as isoprenoids) are a class of hydrocarbons bearing as the base unit an isoprene moiety (i.e. 2-methyl-buta-1,3-diene). Isoprene [CH₂=C(CH₃)CH=CH₂] is represented below (1):

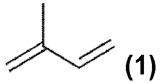

Terpenes may be classified according to the number n (integer) of isoprene units of which it is composed, for example:
n = 2: monoterpenes (C₁₀), such as myrcene or pinene (alpha or beta), are the most common;
n = 3: sesquiterpenes (C₁₅), such as farnesene;
n = 4: diterpenes (C₂₀);
n = 5: sesterpenes (C₂₅);
n = 6: triterpenes (C₃₀), such as squalane;
n = 7: tetraterpenes (C₄₀), such as carotene (C₄₀H₆₄), which is an important pigment of plant photosynthesis.

May isomers exist in each of the families.

The carbon backbone of terpenes may consist of isoprene units arranged end to end to form linear molecules. The arrangement of the isoprene units may be different to form a branched or cyclic backbone.

Terpenes thus include linear isoprene compounds, branched isomers and also cyclic compounds, for instance pinene or carotene derivatives.

When terpenes are chemically modified, for example by oxidation and/or rearrangement of the carbon backbone, the compounds obtained are called terpenoids. The carbon backbone of terpenoids may thus not strictly correspond to the addition of several isoprene units, due to the loss or displacement of a fragment, generally a methyl group.

Terpenoids may be isoprene unit derivatives including one or more oxygen atoms in the functional groups.

Terpenoids may thus be considered as terpenes that have been chemically modified, for example by oxidation and/or rearrangement of the carbon backbone, with one or more fragments (for example methyl group) added or removed, or with at least one oxygen atom added, for instance as in the camphor molecule.

In the present invention, it is thus considered that terpenoids and terpenes are two separate families that do not have compounds in common.

Terpenoids may also be used as molecules serving as charge derived from biomass. However, terpenoids made by addition of a heteroelement to a terpene unit are not included in the category of target molecules. The reason for this is that the presence of a heteroelement would greatly degrade the properties of the jet fuel formed with these molecules.

Only terpenoids made by adding or removing an alkyl group, for example, methyl, are included in the target molecules.

As with terpenes, terpenoids may be classified according to the number of isoprene units of which they are composed: a monoterpenoid comprises two isoprene units, a sesquiterpenoid comprises three isoprene units, a diterpenoid comprises four isoprene units, a sesterpenoid comprises five isoprene units, a triterpenoid comprises six isoprene units, a tetraterpenoid comprises seven isoprene units, etc.

A description of terpenes is available in the "Traité de chimie organique by Vollhardt and Schore (5th edition, page 152)".

In summary, the molecules that can be treated in the process of the invention are thus terpenes and/or terpenoids whose carbon chain is linear, branched or cyclic and comprises n isoprene units, n being between 1 and 12.

The number n of isoprene units will depend on the availability of the biomass for jet fuel applications. The two steps of the process may be repeated to adapt the carbon chain length for jet applications.

The terpenes used may thus comprise from 2 to 12, or even from 2 to 8, from 2 to 7, or even from 2 to 6 isoprene units. The greater the number of units, the more times steps (i) and (ii) will have to be repeated to obtain the correct carbon chain length.

More advantageously, terpenes or terpenoids used include from 2 to 6 isoprene moieties. In such a case, compounds produced according to the invention are thus belonging to C₁₀ to C₃₀ compounds.

The choice of terpenes or terpenoids or mixture thereof as the charge, allows the production of final compounds, in particular a mixture of paraffins and/or naphthenic compounds presenting a carbon chain length from C9 to C14, typically with satisfactory yields and/or also presenting appropriate properties for direct applications as jet fuels. Advantageously, such final compounds do not require any further treatments so as to make them compliant with uses as jet fuels.

Particularly, where in terpenes or terpenoids n is between 2 and 12, preferably from 2 to 8, more preferably from 2 to 7, especially from 2 to 6, best results regarding reaction yields may especially be obtained.

The charge may advantageously be heated before performing the cycloaddition step (i), so as to facilitate the cycloaddition reaction.

The cycloaddition step (i) may also be performed in the presence of a gas that is inert for this reaction.

In particular, gases such as rare gases, dinitrogen (N₂) or dihydrogen (H₂) may be introduced from step (i) without modifying the cycloaddition reaction.

Advantageously, the cycloaddition step (i) may be performed in the presence of dihydrogen (H₂), which is then separated out and reused during the hydrogenation and selective cracking step (ii).

Additional dihydrogen may optionally be introduced before performing the hydrogenation and selective cracking step (ii).

Advantageously, the cycloaddition reaction of step (i), for example according to the Diels-Alder reaction, is performed in the presence of at least one antioxidant compound in order to limit the risks of a runaway reaction. The antioxidant compound is advantageously a stabilizer allowing to limit the radical polymerization side reactions which lead to heavier compounds.

This antioxidant compound may be, for example, BHT (butylated hydroxytoluene or 2,6-bis(1,1-dimethylethyl)-4-methylphenol), TBHP (*tert*-butyl hydroperoxide or 2-methylpropan-2-peroxol), or any other antioxidant compound.

The cycloaddition may be performed via the Diels-Alder reaction.

The Diels-Alder reaction is a common reaction in organic chemistry. It may be performed thermally, solely by heating the compounds to be treated.

The Diels-Alder reaction may be performed at atmospheric pressure and in temperature ranges from room temperature to 140°C.

The reaction may be performed at higher temperature when the products are heavier. The pressure may also be increased. Although the reaction takes place at atmospheric pressure, it may advantageously be brought to the pressure necessary to perform the hydrogenation and selective cracking reaction, for example, to a pressure of 50 to 150 bar. Thus, the cycloaddition and hydrogenation and selective cracking steps may be performed in sequence without intermediate compression.

The Diels-Alder reaction of step (i) may also be performed in the presence of a catalyst (catalytic root), which may make it possible to accelerate the reaction.

The Diels-Alder reaction is a thermal reaction that may be catalyzed with Lewis acid or Bronsted acid catalysts in homogeneous or heterogeneous medium. Catalysts of the type such as AlCl₃ or ZnCl₂ catalyze this reaction. An example of using myrcene in the Diels-Alder reaction is available in the article: Journal of Molecular Catalysis A: Chemical 148 (1999) 87-95.

Other catalysts such as zeolites may be used. Further details regarding these catalysts are available in "March's Advanced Organic Chemistry, fifth edition, M. B. Smith and J. March (Wiley Interscience publication pages 1065 et seq.)".

Other cycloadditions may also be envisaged for performing the cycloaddition step (i), such as (2+2) cycloadditions or trimerizations or tetramerizations. The cycloaddition reactions of the prior art are described in detail in "March's Advanced Organic Chemistry, fifth edition, M. B. Smith and J. March (Wiley Interscience publication pages 1077-1092 et seq.)".

The hydrogenation step and selective cracking step (ii) is performed after the cycloaddition step (i).

These two steps (i) and (ii) may optionally be performed in a single reactor.

The process according to the invention may comprise a fractionation step after the cycloaddition step (i) and before the hydrogenation and selective cracking step (ii), during which the products formed in the cycloaddition step (i) are separated from the unconverted charge before being treated in the hydrogenation and selective cracking step (ii).

Separation of the unreacted charge and of the products of the cycloaddition reaction may be performed by distillation, preferably under vacuum (for example from 1.33 10⁻² to 1.33 10⁻¹ Bar, i.e.10 to 100 millimetres of Hg).

The dihydrogen (H₂) may be separated out by flash distillation.

In particular, the unconverted charge separated out during this fractionation step may then be sent back to the cycloaddition step (i).

In a first variant of the invention, the hydrogenation and selective cracking step (ii) comprises a hydrogenation step and a selective cracking step that are performed separately, the hydrogenation step being performed first.

However, since the cracking reaction is performed under dihydrogen and under harsher conditions than the hydrogenation reaction, hydrogenation of the compounds also takes place during the cracking reaction performed separately.

The hydrogenation reaction of step (ii) may take place on a standard hydrogenation catalyst. By way of example, catalysts of the family of nickel reduced in metallic form may be mentioned.

The catalytic cracking reaction of step (ii) may take place on the catalysts generally used for the hydrocracking of hydrocarbons. Hydrocracking catalysts are bifunctional catalysts, i.e. they bear an acid function and a metallic function enabling the hydrogenation. The acid function is provided by the support. This support generally consists of a porous support with a large specific surface area. Supports such as zeolites, amorphous aluminosilicas or mixed oxides can be used. The hydrogenation reactions are performed on the metallic function. Metals such as noble metals (Pt, Pd, etc.) catalyze hydrogenation reactions. Other metals such as metals from group VIB of the Periodic Table or a combination of metals from group VIB with metals from group VIII (preferably non-noble metals) such as nickel or cobalt may also be used in the preparation of hydrocracking catalysts. By way of example, patent EP 1535983 describes the preparation of a hydrocracking catalyst.

In a second variant of the invention, the hydrogenation and selective cracking step (ii) comprises a hydrogenation step and a selective cracking step that are performed simultaneously. In this case, the metallic function of the cracking catalyst acts as the hydrogenation catalyst.

The hydrogenation and selective cracking reaction of step (ii) may be performed on a hydrocracking catalyst already described above. It may also advantageously be performed on a catalyst prepared on a mesoporous support having shape selectivity.

In this case, when the cycloaddition reaction is a Diels-Alder reaction, this reaction may be either catalytic or thermal. The catalysts that may be used for the Diels-Alder reaction are those already described.

Finally, the selective cracking reaction may be performed on the catalysts described above.

An example of a reaction scheme envisaged for this second variant is shown below starting with myrcene. In a first step, the myrcene dimer (camphorene isomer) is prepared via a Diels-Alder reaction. It is then treated directly on a hydrogenation and cracking catalyst, for example, according to reaction scheme (I):

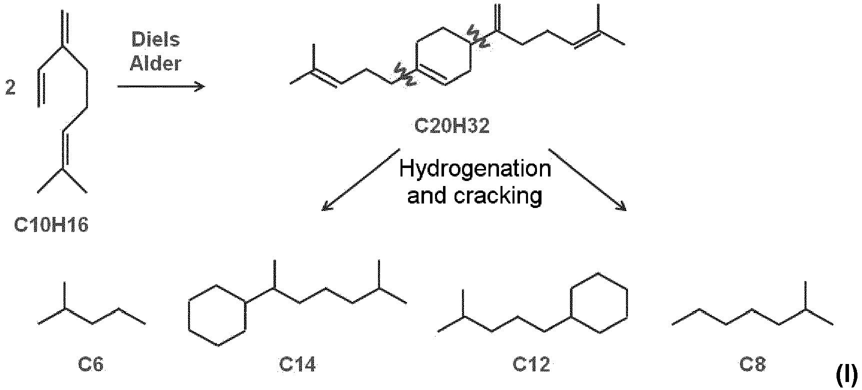

This type of reaction scheme may also be envisaged for longer terpenes such as farnesene, for example according to reaction scheme (II):

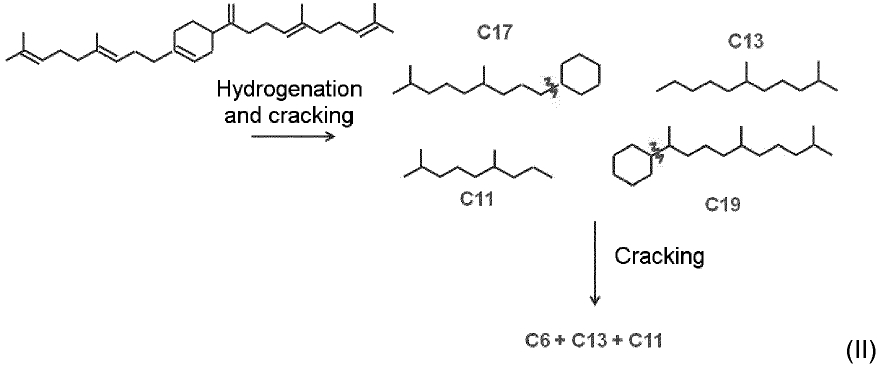

Irrespective of the embodiment, the hydrogenation and selective cracking steps of step (ii) may be performed in the same reactor.

Irrespective of the embodiment, the charges treated in the cycloaddition step (i) and/or the hydrogenation and selective cracking step (ii) may be treated in pure form or diluted in a compound that is inert towards the cycloaddition, hydrogenation and cracking reactions.

Advantageously, the hydrogenation and selective cracking step (ii) is performed in a single reactor.

The use of several reactors may also be envisaged.

The hydrogenation and selective cracking step may thus be performed in the presence of at least one catalyst.

One or more beds comprising at least one catalyst formulated to catalyze the hydrogenation reaction and also the cracking reaction may be used. The hydrogenation catalysts that may be used include catalysts bearing a metallic function in the form of a noble metal (for example Pt, Pd) or alternatively other metallic catalysts such as Ni-based catalysts.

The process according to the invention may also include a fractionation step after performing the cycloaddition step (i) and/or the hydrogenation and selective cracking step (ii). This fractionation step may be performed by flash distillation or by simple distillation.

When fractionation is performed after the cycloaddition step (i), the lightest compounds, i.e. the compounds that have not reacted, are separated out and recycled. The cycloaddition products are removed in order to be treated in step (ii). The heavier products (i.e. the compounds formed by the oligomerization of at least three molecules) that may be formed by polycondensation reactions are separated out and removed. These products may advantageously be retreated. The inert gas used in step (i) may optionally be withdrawn and recycled.

After step (ii), various compounds may be separated. When the charge has been diluted, the diluent may be withdrawn, for example, by distillation. The dihydrogen used for the reaction and which has not reacted may be recycled into step (i) or (ii). Finally, the cyclic compounds that have not been totally cracked, i.e. which bear a carbon chain length containing more than fourteen carbon atoms, may be separated out and advantageously recycled into the start of step (ii). The cracking products, in particular of C9-C14, once separated out may be incorporated directly into a pool of jet fuel bases.

Another subject of the invention relates to a jet fuel comprising at least some of the products derived from the process described previously.

The products formed by the sequence of reactions (i) and (ii) may be incorporated directly into jet fuels: these molecules have the properties of a jet fuel. The incorporation of these molecules into jet fuel bases may be performed in a range from 1 % to 95% by weight relative to the other fuel bases derived from petroleum products. The amount introduced is dependent on the specification to be achieved. By way of example, the volatility of the C14 and C12 molecules obtained by the selective cracking of dimyrcene have a flash point > 38°C (Abel device - ASTM D3822/IP170).

The invention is now described with reference to the attached drawings:

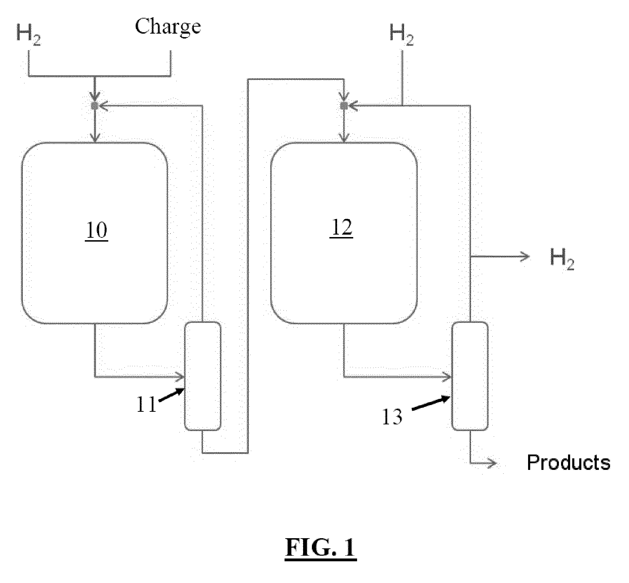

Figure 1 shows an example of a process scheme according to one embodiment of the invention.

The scheme in Figure 1 comprises a cycloaddition reactor 10 for performing the cycloaddition reaction and a hydrogenation and selective cracking reactor 12 for performing the hydrogenation and selective cracking steps.

The charge and the inert gas (for the cycloaddition reaction) are introduced into the cycloaddition reactor 10.

The cycloaddition reaction may be performed in a fixed bed, in a batch reactor, or alternatively in a semi-continuous batch.

The cycloaddition reactor 10 may contain an inert gas for the cycloaddition reaction or a homogenous or heterogeneous catalyst.

At the outlet of the cycloaddition reactor 10, a separator 11 enables recycling of the unreacted compounds and of the hydrogen.

The separation of the unreacted charge and of the products may be performed by vacuum distillation. The H₂ may be separated out by flash before being injected into the hydrogenation and selective cracking reactor 12.

The amount of hydrogen necessary for the hydrogenation and selective cracking reaction is much larger than the amount of hydrogen necessary for coverage of the cycloaddition reaction. The recycling of hydrogen is thus advantageously performed in the reactor 12.

Additional hydrogen may be added before the products obtained from the cycloaddition reactor 10 are treated in the hydrogenation and selective cracking reactor 12, to ensure adequate coverage of hydrogen. The hydrogenation and selective cracking reactions may be performed in a fixed bed, in a batch reactor, or alternatively in a semi-continuous batch. At the outlet of this second reactor 12, a separator 13 enables extraction of the hydrogen and the unreacted products. The latter are recycled into the top of the hydrogenation and selective cracking reactor 12. The separation may be performed by flash or by distillation. The products that have reacted are removed at the bottom of the column.

### Examples

Examples 1 and 2 correspond to the embodiment in which the cycloaddition step (i) is followed by a hydrogenation and selective cracking step (ii) during which the hydrogenation and selective cracking reactions are performed simultaneously.

### Example 1

In a first stage, the Diels-Alder reaction was performed thermally on myrcene.

Myrcene, or 7-methyl-3-methylene-1,6-octadiene, is a monoterpene of empirical formula C₁₀H₁₆ and of structural formula (2):

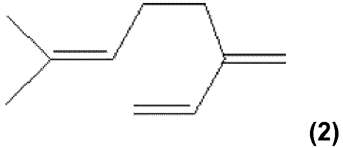

In a reflux assembly, commercial myrcene (commercial source Sigma-Aldrich) was heated to various temperatures. Several reaction times were tested. All the tests were performed under a stream of dinitrogen (N₂).

The results are presented in Table 1.

**Table 1: Results of Example 1**

| Test No. | Temperature | Time | Catalyst | % Conversion calculated by simulated distillation | % Dimyrcene |
|---|---|---|---|---|---|
| 1 | 170°C | 5h | None | 86% | 66% |
| 2 | 100°C | 2h | None | 12% | 3% |
| 3 | 100°C | 2h | AlCl₃ | 10% | 4% |
| 4 | 170°C | 2h30 | None | 75% | 50% |
| 5 | 100°C then 170°C | 2h then 1h | None | 70% | 46% |

Incidentally, commercial myrcene contains 1000 ppm by weight of BHT. This compound serves as a stabilizer and limits polymerization reactions. The use of the stabilizer in the Diels-Alder reaction makes it possible to limit radical polymerization reactions. There is therefore a direct advantage in using commercial myrcene. Not only is no purification necessary to remove the stabilizer, but also the stabilizer makes it possible to limit the radical polymerization side reactions which lead to heavier compounds.

Tests 2 and 3 show only a low conversion of myrcene. The analyses of these samples will therefore not be presented.

The GC-MS analyses revealed the molecular masses of the compounds formed in the samples of tests 1 and 5:
- at retention times 22.2; 22.9 minutes: M = 136 g/mol compound of empirical formula C₁₀H₁₆; myrcene and isomers;
- at retention times 46.6, 47.1, 47.6, 48.3 minutes: M = 272 g/mol C₂₀H₃₂, myrcene dimers (4 isomers identified).

The structural formulae of the various isomers of the compound of empirical formula C₂₀H₃₂ revealed in the Diels-Alder reaction are presented below.

### Isomer 1:

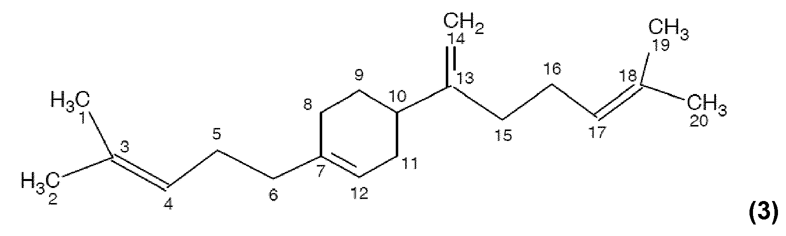

### Isomer 2:

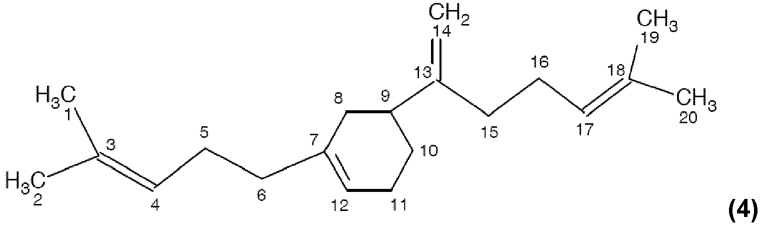

### Isomer 3:

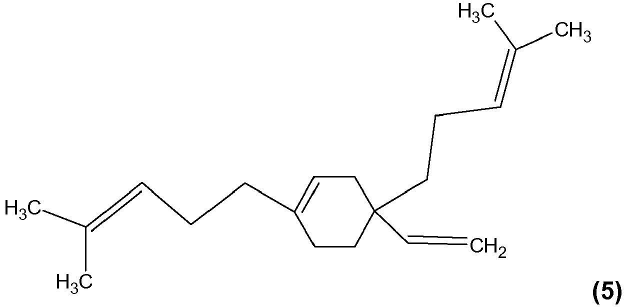

### Isomer 4:

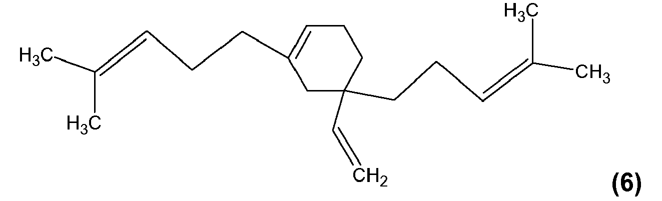

### Impurity present in myrcene (5% by mass):

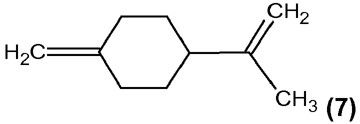

The samples of test 1 were analyzed by NMR. This analysis revealed several dimyrcene isomers. The selectivity of the products obtained is shown in Table 2 below. This selectivity is calculated on the products identified by NMR, i.e. on the dimyrcene isomers and myrcene. The heavy compounds are not identified by NMR.

**Table 2**

| Composition: | Mol% | Molar mass (g/mol) | Mass% |
|---|---|---|---|
| Isomer 2 (C20) | 33 | 272.5 | 38 |
| Isomer 1 (C20) | 26 | 272.5 | 30 |
| Myrcene (C10) | 17 | 136.2 | 10 |
| Impurity (C10) | 11 | 136.2 | 6 |
| Isomer 3 or 4 (C20) | 6 | 272.5 | 7 |
| Isomer 3 or 4 (C20) | 7 | 272.5 | 8 |

Similarly, the various isomers obtained after test 5 are presented in Table 3 below.

**Table 3**

| Composition: | Mol% | Molar mass (g/mol) | Mass% |
|---|---|---|---|
| Isomer 2 (C20) | 19 | 272.5 | 27 |
| Isomer 1 (C20) | 15 | 272.5 | 21 |
| Myrcene (C10) | 49 | 136.2 | 34 |
| Impurity (C10) | 9 | 136.2 | 7 |
| Isomer 3 or 4 (C20) | 4 | 272.5 | 5 |
| Isomer 3 or 4 (C20) | 4 | 272.5 | 6 |

The reaction time and temperature play an important role in the conversion of myrcene into dimyrcene. More dimyrcene may be obtained with heating at 170°C for 5 hours.

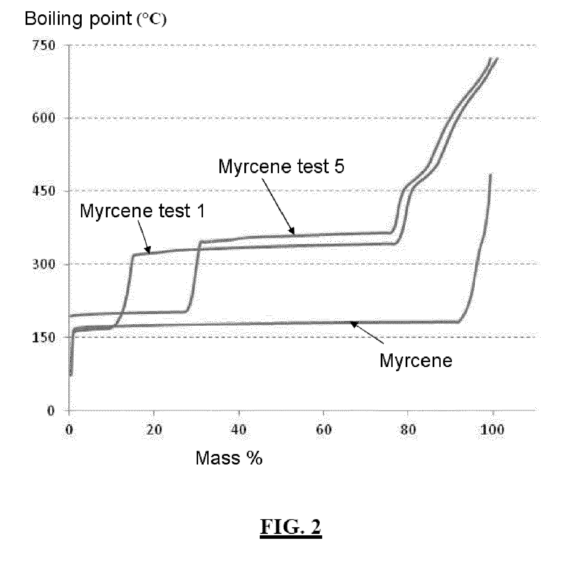

The results of the simulated distillations are presented in Figure 2. Pure myrcene shows a plateau at its boiling point. Once the Diels-Alder reaction has taken place, the myrcene plateau is regained. A plateau corresponding to the dimyrcene isomers is visible at about 330-340°C. Heavy compounds (higher boiling points) are also formed.

### Example 2

The mixture obtained by the Diels-Alder reaction is then purified by vacuum distillation. In a standard distillation assembly (three-necked flask, Vigreux column on which is mounted a condenser), the mixture obtained previously is heated under vacuum (1.33 10⁻² Bar, i.e.10 mmHg). The first fraction contains essentially myrcene (distillation temperature < 200°C). In the second fraction (distillation temperature = 200°C), the dimyrcene isomers are present. The distillation yield is presented in Table 4 below.

**Table 4**

| Compounds | Mass yield for the distillation |
|---|---|
| Myrcene | 25% |
| Myrcene dimers | 47% |
| Heavy molecules (polymyrcene) | 26% |
| Losses | 2% |

The dimers thus formed were analyzed by gas chromatography. Distillation enabled the dimers to be purified to 99.8% by weight.

Once the dimers were separated out, the hydrogenation and selective cracking reactions were performed in a batch reactor under hydrogen pressure.

The main tests performed are summarized in Table 5 below.

**Table 5**

| Catalyst | T (°C) | P (bar) | Test time | Dimers | Selective cracking products | Products derived from unselective cracking and/or isomerized products (C4-C10) | Unknown |
|---|---|---|---|---|---|---|---|
| IrWO₃/Al₂O₃ | 350 | 100 | 24 h | 90.8% | 1.7% | 0.5% | 7.0% |
| Ir-KF200 | 300 | 50 | 4 h | 92.6% | 2.0% | 0.9% | 4.5% |
| Ir-KF200 | 350 | 100 | 12 h | 26.8% | 4.9% | 11.6% | 56.6% |
| Ir-KF200 | 350 | 100 | 24 h | 25.9% | 5.3% | 12.9% | 55.9% |

KF200 is a catalyst prepared from Pt and Pd dispersed on an amorphous aluminosilica. The catalyst used in the tests presented above contains 0.7% by weight of Pt, 0.7% by weight of Pd and 1% by weight of Ir. The products derived from the selective cracking are the following:
- 2-methylpentane
- cyclohexane
- 2-methylheptane
- (4-methylpentyl)cyclohexane.

Other products derived from the cracking may be isomerized and include all the identifiable C₄ to C₁₀ molecules.

By modifying the operating conditions, the nature of the active phase and the nature of the support, it is possible to promote the cracking reaction so as to obtain molecules with a chain length of between C8 and C14. This reaction may advantageously be coupled with a separation and recycling in order to increase the overall yield of C9-C14 cracking products.

### Analyses used in the examples

### NMR

The ¹H and ¹³C-NMR 1D spectra were recorded on a Bruker DRX 500 MHz spectrometer equipped with a BBO 5 mm probe.

In the ¹H observation sequence, the acquisition parameters used are the following:
- Relaxation time of 10 s
- 32k points
- Spectral width of 15 ppm
- 16 scans

The ¹³C spectrum was obtained with an "inverse gated decoupling" pulse sequence (¹H decoupling, no NOE effect) and the following acquisition parameters:
- Relaxation time of 30 s
- 64k points
- Spectral width of 241 ppm
- 180 scans

The spectra were recorded in CDCl₃ (500 µl of sample + 250 µl of solvent) with TMS as internal reference and at room temperature.

The 2D-inadequate spectra (¹³C-¹³C) of the sample on sample 1 of test 1 were recorded on a Bruker Avance III 500 MHz spectrometer equipped with a 10 mm cryoprobe. This analysis made it possible to identify the chemical shifts associated with each of the isomers. Once the shifts were identified, 1D ¹³C spectra were acquired.

The spectrum was recorded in CDCl₃ (2 g of sample + 1 ml of solvent) with TMS as internal reference and at room temperature.

The chemical shifts of the isomers are presented in Tables 6 and 7 below. The carbon numbering is that presented on the structural formula of the isomer.

**Table 6: Chemical shifts for the ¹³C NMR spectrum of Isomer 1**

| **Structural formula of Isomer 1** | |
|---|---|
| 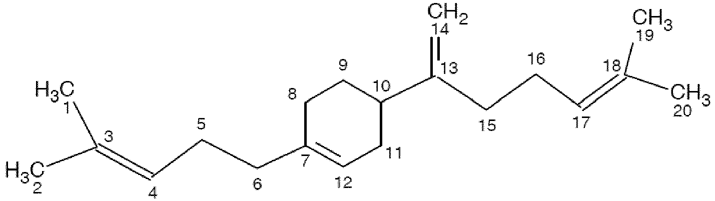 | |
| **Carbon atom number** | **Chemical shift (ppm)** |
| 1 | 17.7 |
| 2 | 25.7 |
| 3 | 131 or 131.2 |
| 4 | 124.5 or 124.6 |
| 5 | 26.6 |
| 6 | 37.8 |
| 7 | 137.2 |
| 8 | 29.2 |
| 9 | 28.5 |
| 10 | 40.1 |
| 11 | 31.5 |
| 12 | 120.5 |
| 13 | 154 |
| 14 | 107.3 |
| 15 | 35 |
| 16 | 27 |
| 17 | 124.5 or 124.6 |
| 18 | 131 or 131.2 |
| 19 | 17.7 |
| 20 | 25.7 |

**Table 7: Chemical shifts for the ¹³C NMR spectrum of Isomer 2**

| **Structural formula of Isomer 2** | |
|---|---|
| 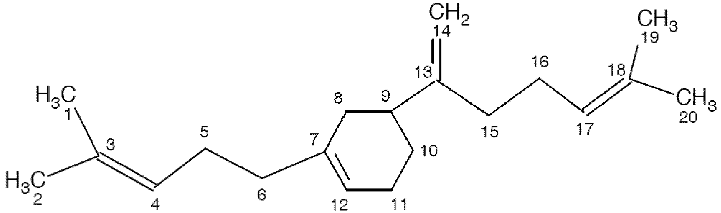 | |
| **Carbon atom number** | **Chemical shift (ppm)** |
| 1 | 17.7 |
| 2 | 25.7 |
| 3 | 131 or 131.2 |
| 4 | 124.5 or 124.6 |
| 5 | 26.6 |
| 6 | 38 |
| 7 | 137.4 |
| 8 | 34.7 |
| 9 | 40.5 |
| 10 | 28.1 |
| 11 | 26 |
| 12 | 120.3 |
| 13 | 153.9 |
| 14 | 107.4 |
| 15 | 35 |
| 16 | 27 |
| 17 | 124.5 or 124.6 |
| 18 | 131 or 131.2 |
| 19 | 17.7 |
| 20 | 25.7 |

### Gas chromatography and mass spectrometry

The chromatographs were acquired on an HP6890 chromatograph. The column used is an apolar PONA capillary column (50 m/0.2 mm/0.5 µ). The injection of 0.5 µl was performed with an automatic injector. The column flow rate is 0.7 ml/min (constant flow rate). The injector is at a temperature of 250°C with a split ratio of 147. Programme: 50°C isotherm for 5 minutes; then from 50°C to 300°C at 5°C/min; isotherm at 300°C for 30 minutes. The sample was diluted in hexane for the Diels-Alder reaction. The sample was diluted in heptane as regards the selective cracking tests. The quantifications were acquired with an FID detector.

The mass spectrometry detector used is an MSD5975, 26-600 detector.

### Simulated distillation

The simulated distillation curves were determined by gas chromatography according to standard ASTM D 7169 adapted for heavy petroleum products.

## Claims

1. Process for preparing jet fuel or jet fuel precursors which includes the treatment of a charge derived from biomass, said charge comprising at least one compound chosen from
- terpenes of formula [CH₂=C(CH₃)CH=CH₂]n, in which n is an integer of from 2 to 12, the carbon-based chain of which is linear, cyclic or branched, or
- terpenoids which are cyclic or branched terpenes as defined previously, which have been chemically modified by oxidation and/or rearrangement of the carbon backbone with an alkyl group added or removed,
said process comprising a cycloaddition step (i) followed by a hydrogenation and selective cracking step (ii).

2. Preparation process according to Claim 1, which comprises a fractionation step between the cycloaddition step (i) and the hydrogenation and selective cracking step (ii), during which the products formed in the cycloaddition step (i) are separated from the unconverted charge before being treated in the hydrogenation and selective cracking step (ii).

3. Preparation process according to Claim 2, in which the unconverted charge separated out during the fractionation step is sent back to the cycloaddition step (i).

4. Preparation process according to one of Claims 1 to 3, in which the hydrogenation and selective cracking step (ii) comprises a hydrogenation step and a selective cracking step that are performed separately, the hydrogenation step being performed first.

5. Preparation process according to one of Claims 1 to 3, in which the hydrogenation and selective cracking step (ii) comprises a hydrogenation step and a selective cracking step that are performed simultaneously.

6. Preparation process according to one of Claims 1 to 5, in which the hydrogenation and selective cracking step (ii) is performed in the presence of at least one bifunctional hydrocracking catalyst, bearing an acid function and a metallic function enabling hydrogenation.

7. Preparation process according to one of Claims 1 to 6, in which the hydrogenation and selective cracking step (ii) is performed in a single reactor.

8. Preparation process according to one of Claims 1 to 7, which includes a fractionation step after performing the hydrogenation and selective cracking step (ii).

9. Preparation process according to Claim 8, in which the cyclic compounds with a carbon chain length containing more than fourteen carbon atoms are recycled into the start of step (ii).

10. Preparation process according to one of Claims 1 to 9, in which the cycloaddition step is a cycloaddition according to the Diels-Alder reaction.

11. Preparation process according to one of Claims 1 to 10, in which the alkyl group added or removed from terpenes to make terpenoids is a methyl group.

## Patentansprüche

1. Verfahren zur Herstellung von Düsentreibstoff oder Düsentreibstoffvorläufern, das die Behandlung eines Einsatzmaterials, abgeleitet von Biomasse, beinhaltet, wobei das Einsatzmaterial mindestens eine Verbindung, ausgewählt aus
- Terpenen der Formel [CH₂=C(CH₃)CH=CH₂]n, in welcher n eine ganze Zahl von 2 bis 12 ist, wobei die kohlenstoffbasierte Kette von diesen linear, ringförmig oder verzweigt ist, oder
- Terpenoiden, die ringförmige oder verzweigte Terpene wie vorstehend definiert darstellen, welche durch Oxidation und/oder Umlagerung der Kohlenstoffhauptkette chemisch mit einer hinzugefügten oder entfernten Alkylgruppe modifiziert wurden,
umfasst,
wobei das Verfahren einen Cycloadditionsschritt (i), gefolgt von einem Hydrierungs- und selektiven Crackschritt (ii) umfasst.

2. Herstellungsverfahren nach Anspruch 1, das einen Fraktionierungsschritt zwischen dem Cycloadditionsschritt (i) und dem Hydrierungs- und selektiven Crackschritt (ii) umfasst, während dem die in dem Cycloadditionsschritt (i) gebildeten Produkte von dem nicht umgewandelten Einsatzmaterial getrennt werden, bevor sie in dem Hydrierungs- und selektiven Crackschritt (ii) behandelt werden.

3. Herstellungsverfahren nach Anspruch 2, in dem das während des Fraktionierungsschritts abgetrennte, nicht umgewandelte Einsatzmaterial in den Cycloadditionsschritt (i) zurückgeführt wird.

4. Herstellungsverfahren nach einem der Ansprüche 1 bis 3, in dem der Hydrierungs- und selektive Crackschritt (ii) einen Hydrierungsschritt und einen selektiven Crackschritt, die getrennt voneinander durchgeführt werden, umfasst, wobei der Hydrierungsschritt zuerst durchgeführt wird.

5. Herstellungsverfahren nach einem der Ansprüche 1 bis 3, in dem der Hydrierungs- und selektive Crackschritt (ii) einen Hydrierungsschritt und einen selektiven Crackschritt, die gleichzeitig durchgeführt werden, umfasst.

6. Herstellungsverfahren nach einem der Ansprüche 1 bis 5, in dem der Hydrierungs- und selektive Crackschritt (ii) in der Gegenwart von mindestens einem bifunktionellen Hydrocrackkatalysator, der eine Säurefunktion und eine Hydrierung ermöglichende metallische Funktion trägt, durchgeführt wird.

7. Herstellungsverfahren nach einem der Ansprüche 1 bis 6, in dem der Hydrierungs- und selektive Crackschritt (ii) in einem Einzelreaktor durchgeführt wird.

8. Herstellungsverfahren nach einem der Ansprüche 1 bis 7, das einen Fraktionierungsschritt nach dem Durchführen des Hydrierungs- und selektiven Crackschritts (ii) enthält.

9. Herstellungsverfahren nach Anspruch 8, in dem die ringförmigen Verbindungen mit einer Kohlenstoffkettenlänge, die mehr als vierzehn Kohlenstoffatome enthält, an den Anfang von Schritt (ii) zurückgeführt werden.

10. Herstellungsverfahren nach einem der Ansprüche 1 bis 9, in dem der Cycloadditionsschritt eine Cycloaddition gemäß der Diels-Alder-Reaktion ist.

11. Herstellungsverfahren nach einem der Ansprüche 1 bis 10, in dem die Alkylgruppe, die den Terpenen hinzugefügt oder von diesen entfernt wurde, um Terpenoide herzustellen, eine Methylgruppe ist.

## Revendications

1. Procédé de préparation de jet fuel ou de précurseurs de jet fuel lequel comprend le traitement d'une charge issue de la biomasse, ladite charge comprenant au moins un composé choisi parmi :
- les terpènes de formule [CH₂=C(CH₃)CH=CH₂]n, où n est un entier de 2 à 12, dont la chaîne carbonée est linéaire, cyclique ou ramifiée, ou
- les terpénoïdes qui sont des terpènes tels que définis précédemment, qui ont été modifiés chimiquement par oxydation et/ou réarrangement du squelette carboné, cycliques ou ramifiés par ajout ou retrait d'un groupement alkyle,
ledit procédé comprenant une étape (i) de cycloaddition suivie d'une étape (ii) d'hydrogénation et de craquage sélectif.

2. Procédé de préparation selon la revendication 1, lequel comprend une étape de fractionnement entre l'étape (i) de cycloaddition et l'étape d'hydrogénation et de craquage sélectif (ii), au cours de laquelle les produits formés au cours de l'étape (i) de cycloaddition sont séparés de la charge non convertie avant d'être traités par l'étape d'hydrogénation et de craquage sélectif (ii).

3. Procédé de préparation selon la revendication 2, dans lequel la charge non convertie séparée lors de l'étape de fractionnement est renvoyée à l'étape de cycloaddition (i).

4. Procédé de préparation selon l'une des revendications 1 à 3, dans lequel l'étape d'hydrogénation et de craquage sélectif (ii) comprend une étape d'hydrogénation et une étape de craquage sélectif mises en oeuvre séparément, l'étape d'hydrogénation étant mise en oeuvre en premier.

5. Procédé de préparation selon l'une des revendications 1 à 3, dans lequel l'étape d'hydrogénation et de craquage sélectif (ii) comprend une étape d'hydrogénation et une étape de craquage sélectif mises en oeuvre simultanément.

6. Procédé de préparation selon l'une des revendications 1 à 5, dans lequel l'étape d'hydrogénation et de craquage sélectif (ii) est mise en oeuvre en présence d'au moins un catalyseur d'hydrocraquage bi-fonctionnel, possédant une fonction acide et une fonction métallique permettant l'hydrogénation.

7. Procédé de préparation selon l'une des revendications 1 à 6, dans lequel l'étape (ii) d'hydrogénation et de craquage sélectif est mise en oeuvre dans un unique réacteur.

8. Procédé de préparation selon l'une des revendications 1 à 7, lequel comprend une étape de fractionnement après la mise en oeuvre de l'étape d'hydrogénation et craquage sélectif (ii).

9. Procédé de préparation selon la revendication 8, dans lequel les composés cycliques présentant une longueur de chaîne carbonée contenant plus de quatorze atomes de carbone sont recyclés en tête de l'étape (ii).

10. Procédé de préparation selon l'une des revendications 1 à 9, dans lequel l'étape de cycloaddition est une cycloaddition selon la réaction de Diels Alder.

11. Procédé de préparation selon l'une des revendications 1 à 10, dans lequel le groupement alkyle ajouté ou retiré des terpènes pour obtenir les terpénoïdes est un groupement méthyle.
